# NEUE EUROPÄISCHE PATENTSCHRIFT

(11) **EP 2 076 300 B2**
(45) Veröffentlichungstag und Bekanntmachung des Hinweises auf die Entscheidung über den Einspruch: **13.04.2016**
(45) Hinweis auf die Patenterteilung: 23.11.2011
(21) Anmeldenummer: 07818447.0
(22) Anmeldetag: 26.09.2007
(51) Int. Cl.: A61M 5/24, A61M 5/145, A61M 5/315, G01D 5/34, G01F 23/292

(54) **OPTISCHES BESTIMMEN DER STOPFENPOSITION IN GLASAMPULLEN**
OPTICAL DETERMINATION OF THE POSITION OF THE STOPPER IN GLASS AMPOULES
DÉTERMINATION OPTIQUE DE L'EMPLACEMENT D'UN BOUCHON DANS DES AMPOULES EN VERRE

(30) Priorität: 07.10.2006 DE 102006047537
(43) Veröffentlichungstag der Anmeldung: 08.07.2009
(73) Patentinhaber: Sanofi-Aventis Deutschland GmbH, 65929 Frankfurt am Main (DE)
(72) Erfinder: RICHTER, René, 01062 Dresden (DE)
(74) Vertreter: Epping - Hermann - Fischer
(86) Internationale Anmeldenummer: PCT/EP2007/008363
(87) Internationale Veröffentlichungsnummer: WO 2008/040479

(56) Entgegenhaltungen:
- EP-A1- 1 563 859
- EP-B- 0 858 349
- WO-A-2004/009163
- WO-A1-2004/009163
- DE-A1- 10 236 669
- DE-A1- 19 643 813
- DE-A1- 19 643 813
- DE-U1- 29 908 111
- DE-U1- 29 908 111
- US-A- 4 498 843
- US-A- 4 699 186
- US-A- 4 699 186
- US-A- 5 662 612
- US-A- 5 662 612
- US-A- 6 113 578
- US-B2- 7 033 338

## Beschreibung

Die Erfindung betrifft ein Verfahren zur Bestimmung der Stopfenposition einer Medikamentenampulle in einem medizinischen Gerät mittels einer Lichtquelle und lichtempfindlichen Sensoroberfläche.

Viele Arzneimittel müssen in den Körperinjiziert werden. Dies gilt vor allem für solche, die bei oraler Gabe inaktiviert werden oder an Wirksamkeit entscheidend verlieren. Zu diesen Arzneimitteln zählen insbesondere Proteine (wie z. B. Insulin, Wachstumshormone, Interferone), Kohlehydrate (z. B. Heparin), Antikörper oder die meisten Impfstoffe. Zur Injektion in den Körper werden überwiegend Spritzen, Medikamentenstifte (Pens) oder Medikamentenpumpen verwendet.

Das klassische Injektionsgerätfür Insulin ist die Insulinspritze. Diese findet seit Beginn der Insulintherapie Anwendung, ist aber in den letzten Jahren vor allem in Deutschland durch Einführung der Insulinpens schrittweise verdrängt worden. Dennoch sind heute die Spritzen, z. B. bei Verlust oder Defekt eines Insulinpens, unersetzlich und werden von vielen Diabetikern in Kombination mit Insulinpens verwendet. Besonders bei Reisen ist die Wartungsfreiheit und die weitweite Verfügbarkeit vorteilhaft.

Insulinspritzen unterscheiden sich in ihrer Bezeichnung und Skalierung nach der Konzentration des zu verwendenden Insulin U40 bzw. U100. Das Insulin kann sowohl aus Fläschchen als auch aus den vorgefüllten Ampullen für Insulinpens entnommen werden. Dies ermöglicht das Mischen von verschiedenen Insulinsorten und reduziert die Anzahl der notwendigen Injektionen. Bei dem Aufziehen der Spritze mit Insulin ist besonders auf Blasenfreiheit zu achten. Die direkt sichtbare, aufgezogene Insulindosis ermöglicht dem Anwender eine leichte Kontrolle überdie injizierte Insulinmenge. Füreine fehlerfreie Applikation erfordern Insulinspritzen dennoch Geschick und regelmäßige Anwendung.

Ein weiteres mittlerweile weltweit und insbesondere in Europa sehr verbreitetes Injektionsgerät ist der Insulinpen (Insulinstift).

Dieses schreibstiftgrosse Medizingerät wurde Mitte der 80er Jahre entwickelt und kommt hauptsächlich bei der intensivierten Insulintherapie zum Einsatz. Eine wesentliche Neuerung zu Insulinspritzen ist der Einsatz eines wechselbaren Medikamentenbehälters. Dieser Behälter, auch Karpulle bzw. Ampulle genannt, wird vom Hersteller mit dem Insulin befüllt ausgeliefert und vor Gebrauch in den Insulinpen eingesetzt. Bei Inbetriebnahme des Pens durchsticht eine Nadel die Dichtscheibe der Ampulle und realisiert bei der Applikation des Insulins die parenterale Injektion der vorgewählten Dosis. Ein Injektions- und Auslösemechanismus generiert während der Injektion einen Injektionshub, der den Vorschub eines Kolbens bzw. Stopfens in der Ampulle bewirkt und die Abgabe der vorgewählten Dosis in das Zielgewebe bedingt. Der Mechanismus besteht meist aus einer starren Kolbenstange mit einer dem Ampullenstopfenhub entsprechenden Baulänge.

Insulinpens werden in wegwerfbare ("disposable") und mehrfach verwendbare ("reusable") eingeteilt. Bei wegwerfbaren bilden die Ampulle und die Dosiermechanik eine vom Hersteller vorgefertigte Einheit und werden nach Entleerung der Ampulle gemeinsam entsorgt. Eine Wiederverwendung der Dosiermechanik ist nicht vorgesehen. Im Gegensatz zu den Fertigpens stellen mehrfach verwendbare Pens erhöhte Anforderungen an den Anwender. So muss bei Wechsel der Ampulle die Kolbenstange in die Startposition zurückgesetzt werden. Dies geschieht modellabhängig durch des Drehen bzw. Schieben der Kolbenstange bei gleichzeitiger Aktivierung einer Sonderfunktion in der Dosiermechanik. Dies muss der Anwender sehr sorgfältig durchführen, da aufgrund des täglichen Einsatzes und der hohen mechanischen Belastungen mitunter Fehlfunktionen, z. B. ein Verklemmen der Kolbenstange, auftreten können.

Mehrfach verwendbare Insulinpens werden weiterhin unterteilt in manuelle und halbautomatische Pens. Bei manuellen Pens betätigt der Anwender mit Fingerkraft den Injektionsknopf und bestimmt so Dauer und Verlauf der Injektion. Bei halbautomatischen Insulinpens hingegen wird vor Benutzung manuell eine Feder gespannt, die die notwendige Injektionsenergie speichert. Bei dem eigentlichen Injektionsvorgang wird die Feder vom Anwender entriegelt. Die Injektionsgeschwindigkeit ist durch die Federkraft festgelegt und kann nicht an persönliche Bedürfnisse angepasst werden.

In der WO 2004 009 163 wird ein optischer Sensor zur Verwendung in einem Medikamentenverabreichungssystem offenbart, mittels dessen die Verschiebung der Kolbenstange anhand von transparenten oder reflektierenden Markierungen möglich ist. Als Lichtquelle dient eine Anordnung von LEDs, während als Bildelemente lineare oder zweidimensionale CCD-Elemente eingesetzt werden.

Die EP 858349 B1 offenbart eine Vorrichtung zum optischen Messen und elektronischen Aufzeichnen einer Dosis mit einer Lichtquelle und einem optischen Detektor. Der Detektor ist so angeordnet, dass er die Gesamtmenge des Lichts erfasst, das von einer Spritze reflektiert wird, wobei die reflektierende Lichtmenge im Verhältnis zur Flüssigkeitsmenge in der Spritze steht.

Die WO 2001 566 35 offenbart ein Gerät zur Verabreichung eines Medikaments enthaltend ein Sensorelement, mit welchem ein Erkennungselement und/ oder der Betriebszustand eines Behälters (z.B. die Einschubtiefe eines Kolbens) erkannt werden kann.

Die Erfindung betrifft ein Verfahren zur Bestimmung der Position eines Stopfens einer Medikamentenampulle in einem medizinischen Gerät entlang einer Wegstrecke, gemäß Anspruch 1.

Eine Datenverarbeitungseinheit besteht zum einen aus Hardwarekomponenten wie insbesondere einerzentralen Recheneinheit, einem odermehreren Speichern, Ausgabe- und Steuervorrichtungen sowie den technischen Verbindungen zwischen diesen Teilen, und zum anderen aus Softwarekomponenten wie insbesondere einem Betriebssystem sowie einem Programm zur Steuerung und Auswertung der Durchführung einer Positionsbestimmung.

Das Verfahren bezieht sich in einer Ausführungsform auf die Bestimmung der Position des Stopfens einer Ampulle für ein Arzneimittel und insbesondere auf die Bestimmung der Position des Stopfens einer Insulinampulle. Die Bewegung des Stopfens einer Ampulle für ein Arzneimittel wie beispielsweise Insulin innerhalb eines medizinischen Geräts (z. b. Insulinpen, Insulinpumpe) korrespondiert mit der abgegebenen Menge des Arzneimittels. Die Bestimmung der Position des Stopfens einer Ampulle im Betrieb eines medizinischen Gerätes dient deshalb der Steuerung und Überwachung der abgegebenen Menge des Arzneimittels (z. B. Insulin).

Das Verfahren bezieht sich in einer weiteren Ausführungsform, die nicht Gegenstand der Ansprüche ist, auf die Bestimmung der Position eines Bauteils, welches Teil der Einstellvorrichtung, mittels derer die abzugebende Menge eines Arzneimittels voreingestellt wird, ist. Dieses Bauteil, welches Teil der Einstellvorrichtung ist, kann aus einem geometrisch geformten (rund, oval, quadratisch, rechteckig, sternförmig, mischförmig und andere) und mit der Einstellvorrichtung fest verbundenen Vorsprung bestehen, der sich beim Einstellvorgang zusammen mit der Einstellvorrichtung bewegt und mittels der korrespondierenden Bewegung des Schattenbildes entlang der Sensoroberfläche durch die Datenverarbeitungseinheit in die abzugebende Menge eines Arzneimittels (z. B. Insulin) umgerechnet wird. Die Einstellvorrichtung ist dann die Halterung, welche das Bauteil längs einer Wegstrecke beweglich fixiert. Das Verfahren bezieht sich in einer weiteren Ausführungsform, die ebenfalls nicht Gegenstand der Ansprüche ist auf die Bestimmung der Position einer Vorschubeinrichtung zur Ableitung eines Arzneimittels aus einer Ampulle. Eine Vorschubeinrichtung setzt die vorher eingestellte abzugebende Menge eines Arzneimittels in eine Bewegung eines anderen Bauteils zur Abgabe des Arzneimittels um. Die Vorschubeinrichtung ist einerseits direkt oder indirekt mit dem Stopfen der Arzneimittelampulle und andererseits direkt oder indirekt sowohl mit dem Vorstellmechanismus und dem Auslösemechanismus zur Abgabe des Arzneimittels verbunden. Die Bestimmung der Position der Vorschubeinrichtung im erfindungsgemäßen Verfahren kann durch ein mit der Vorschubeinrichtung fest verbundenes Element (z.B. geometrisch geformter dreidimensionaler Körperoderflächiges Plättchen), wodurch ein entsprechendes Schattenbild auf der Sensoroberfläche erzeugt wird, erfolgen. In diesem Fall ist die Vorschubvorrichtung die Halterung, welche das Bauteil längs einer Wegstrecke beweglich fixiert.

Zur Durchführung des Verfahrens wird in einer weiteren Ausführungsform eine Datenverarbeitungseinheit verwendet, welche im medizinischen Gerät integriert d.h. Bestandteil des medizinischen Gerätes ist. Bei einer weiteren Ausführungsform des Verfahrens wird die Datenverarbeitungseinheit separat betrieben. Für eine solche separat betriebene Datenverarbeitungseinheit kann beispielsweise ein PC, auf welchem ein zum Betrieb des erfindungsgemäßen Verfahrens geeignetes Programm installiert ist, verwendet werden. Der Austausch der Daten und Steuersignale zwischen medizinischem Gerät und Datenverarbeitungseinheit hat dann über geeignete Verbindungswege wie insbesondere Kabelverbindungen, Funkverbindungen oder über Datenträger zu erfolgen.

Bei einer weiteren Ausführungsform des Verfahrens ist zwischen der Lichtquelle und der Halterung, welche das Bauteil längs einer Wegstrecke beweglich fixiert und/oder der Halterung, welche das Bauteil längs einer Wegstrecke beweglich fixiert und der lichtempfindlichen Sensoroberfläche eine Blende und/oder eine Linse (zur Fokusierung oder Zerstreuung) angebracht.

Die Lichtquelle besteht in einer bevorzugten Ausführungsform des Verfahrens aus einer LED-Zeile. Die LED-Zeile kann dabei diffus strahlen. Die einzelnen LED's der LED-Zeile können einen geringen Öffnungswinkel aufweisen. In einer weiteren Ausführungsform kann zwischen der LED-Zeile und dem Bauteil, dessen Position bestimmt werden soll bzw. der Halterung, welche das Bauteil längs einer Wegstrecke beweglich fixiert eine Sammellinse insbesondere eine Zylinderlinse angebracht sein.

In einer weitern Ausführungsform des Verfahrens wird durch die Lichtquelle rotes Licht erzeugt. In einer anderen Ausführungsform des Verfahrens wird durch mindestens 2 nebeneinander ausgerichtete Lichtquellen Laserlicht erzeugt. Dieses Laserlicht kann insbesondere rot sein.

In einer anderen Ausführungsform des Verfahrens besteht die Sensoroberfläche aus einer Reihe angeordneter Sensorelemente, wie z. B. einer CCD-Zeilen-kamera. Die Anordnung erfolgt beispielsweise längs einer Strecke oder in Form einer quadratischen oder rechteckigen Fläche. Diese Sensorelemente können eine wellenlängenabhängige Empfindlichkeit aufweisen, die insbesondere bei rotem Licht am größten ist.

Die Erfindung betrifft weiterhin eine Vorrichtung zur Durchführung eines erfindungsgemäßen Verfahrens in einer oder mehrerer der Ausführungsformen wie zuvor beschrieben, wobei diese Vorrichtung mindestens enthält
a) eine Lichtquelle (z. B. eine LED-Zeile normal oder diffus strahlend; oder die einzelnen LED's mit normalen, großen oder geringem Öffnungswinkel; oder die Lichtquelle weißes, blaues oder rotes Licht erzeugend; die Lichtquelle Laserlicht in weißer, blauer oder roter Farbe erzeugend u.a.) und
b) eine Halterung, welche ein Bauteil (z. B. Stopfen einer Arzneimittelampulle; oder Teil der Dosiervorrichtung eines medizinischen Gerätes insbesondere eines Insulinpen; oder Teil der Vorschubeinrichtung eines medizinischen Geräts insbesondere eine Insulinpen u.a.) beweglich längs einer Wegstrecke fixiert und
c) eine lichtempfindliche Sensoroberfläche (z.B. längs in Reihe oder flächig angeordnete Sensorelemente (z.B. CCD-Zeilenkamera); oder Sensorelemente mit wellenlängenabhängiger Empfindlichkeit; oder Empfindlichkeit am größten bei weisen, blauen oder rotem Licht) und
d) eine Datentverarbeitungseinheit (z. B. aus einer Eingabeeinheit zum Aufnehmen der Daten, zentraler Recheneinheit, einem oder mehreren Speicherelementen, Ausgabeeinheit zum Abgeben der Steuersignale sowie Verbindungselementen der einzelnen Teile samt Betriebsprogramm sowie Programm zur Auswertung der Sensoroberfläche bezüglich Auftretens eines Schattenbildes und der Feststellung der Position des korrespondierenden Bauteils sowie Auswertung bezüglich derzeitlich abhängigen Veränderung des Schattenbildes entlang der Sensoroberfläche und der Feststellung der Position sowie Bewegung des korrespondierenden Bauteils).

Die Datenverarbeitungseinheit kann integrierter Bestandteil des medizinischen Geräts sein oder separat vom medizinischen Gerät betrieben werden. Die integrierte Datenverarbeitungseinheit enthält dabei ein Betriebssystem sowie ein Programm zur Durchführung der erfindungsgemäßen Positionsbestimmung. Wenn die Datenverarbeitungseinheit separat vom medizinischen Gerät betrieben wird, erfolgt der Austausch der Daten und Steuersignale zwischen medizinischem Gerät und Datenverarbeitungseinheit über geeignete Verbindungen wie insbesondere Kabelverbindungen, Funkverbindungen oder mittels Bewegung von Datenträgern. Als separat betriebene Datenverarbeitungseinheit eignet sich insbesondere ein PC mit darauf installiertem Betriebssystem und einem Programm zur Durchführung der erfindungsgemäßen Positionsbestimmung.

Eine erfindungsgemäße Vorrichtung in einer odermehrerderAusführungsformen wie zuvorbeschrieben kann zum Zusammenbau eines medizinischen Gerätes, welches zur Verabreichung eine Arzneimittels (z. B. Insulin, Heparin, Wachstumshormon, Interferon, Impfstoff, Antikörper u. a.) in den menschlichen oder tierischen Körper unter Umgehung des gastro-intestinalen Trakts geeignet ist, verwendet werden.

Die Erfindung bezieht sich außerdem auf ein medizinisches Gerät zur Injektion eines Arzneimittels (z.B. ein Insulin) in den menschlichen Körper, das medizinische Gerät umfassend unter anderem
a) einen Grundkörper zur Montierung von mindestens einem technischen Bauteil;
b) ein technisches Bauteil in Gestalt eines Aufnahmebehälters für ein Arzneimittel (z. B. Ampulle), wobei der Aufnahmebehälter zum einen eine obere Öffnung, die durch einen Stopfen, welcher mit einer Kolbenstange funktionell verbunden ist, fluiddicht verschlossen ist und zum anderen eine untere Öffnung, die mit einer Kanüle verbunden ist und durch welche ein Stoff mittels Bewegung des Stopfens durch die Kolbenstange aus dem Vorratsbehälter gedruckt werden kann;
c) ein technisches Bauteil in Gestalt eines Vorschubmechanismus umfassend zum einen eine Kolbenstange, die mit dem Stopfen direkt oder indirekt verbunden ist, und zum anderen eine Vorschubeinheit, welche nach Betätigung einer Auslösung, die mittels Dosiervorrichtung (z. B. durch Festlegung eines Drehwinkels) voreingestellte Menge der zu dosierenden Menge des Arzneimittels in eine entsprechende Bewegung der Kolbenstange und des Stopfens überführt;
d) ein technischen Bauteil in Gestalt einer Dosiervorrichtung zur Voreinstellung der zu dosierenden Menge des Arzneimittels,
e) ein technisches Bauteil in Gestalt einer Anzeige (mechanische oder LCD Anzeige) zur Wiedergabe der durch die Dosiereinrichtungvoreingestellten und durch das medizinische Gerät zu verabreichenden Menge des zu injizierenden Stoffes;
f) ein technisches Bauteil in Gestalt eines Auslösemechanismus zum Ingangsetzen und Durchführen der Injektion umfassend hierbei auch die Entfernung von Luftblasen aus der Ampulle vor Durchführung der Injektion, gekennzeichnet dadurch, dass zusätzlich eine erfindungsgemäße Vorrichtung in einer oder mehrerer der Ausführungsformen wie zuvor beschrieben enthalten ist.

Ein solches medizinisches Gerät liegt in einer Ausführungsform insbesondere in Form und Funktion eines Insulinpen oder eines Pens geeignet zur Injektion eines anderen Arzneimittels (z. B. Heparin, Wachstumshormon, Interferon, Impfstoff, Antikörper u. a.) vor.

In einerweiteren Ausführungsform umfasst ein solches medizinisches Gerät mindestens ein Mittel zur Speicherung und/oder Verarbeitung von Daten und/oder Signalen sowie mindestens eine Schnittstelle zur Übertragung von Daten und/oder Signalen zu und/oder von einer externen technischen Einheit, welche zur Speicherung und/oder Verarbeitung von Daten und/oder Signalen ausgelegt ist. Solche Mittel bzw. Schnittstellen können unter anderem insbesondere in der Kappe des medizinischen Geräts zur Verfügung gestellt werden.

Die genannte externe technische Einheit kann aus einem PC bestehen, auf welchem ein Programm zur Speicherung und/oder Verarbeitung von Daten und/oder Signalen installiert ist.

Ein medizinisches Gerät in einer oder mehrerer der beschriebenen Ausführungsformen kann zur Injektion von Insulin (normal wirkendes lang wirksames; kurz wirksames) oder von GLP-1 oder Lovenox oder von einem anderen Stoff verwendet werden.

Die Erfindung betrifft weiterhin die Herstellung eines medizinischen Geräts in einer oder mehrerer der beschriebenen Ausführungsformen zur Injektion eines Arzneimittels in den menschlichen odertierischen Körper wobei
a) ein Grundkörper zur Montierung von mindestens einem technischen Bauteil bereitgestellt wird;
b) ein Aufnahmebehälter (z. B. Ampulle für ein Medikament insbesondere Insulin, Heparin, GLP-1 Peptid-Hormon, Wachstumshormon, Lovenox, Impfstoff, Antikörper u. a.) bereitgestellt wird;
c) eine Kolbenstange bereitgestellt wird;
d) ein Vorschubmechanismus bereitgestellt wird;
e) eine Dosiervorrichtung bereitgestellt wird;
f) eine Anzeige bereitgestellt wird;
g) ein Auslösemechanismus bereitgestellt wird;
h) evtl. elektronische Bestandteile (z. B. ein Mittel zur Speicherung und/oder Verarbeitung von Daten und/oder Signalen zu und/oder von einer technischen Einheit z. B. einem PC, welche zur Speicherung und/oder Verarbeitung von Daten und/oder Signalen ausgelegt ist) bereitgestellt werden;
i) eine erfindungsgemäße technische Vorrichtung in einer oder mehrerer der Ausführungsformen wie zuvor beschrieben bereitgestellt wird;
j) die Einzelbestandteile aus a) bis i) zu einer funktionellen Einheit zusammengebaut werden.

Das erfindungsgemäße medizinische Gerät kann insbesondere zur Prophylaxe und/oder Therapie einer Krankheit und/oder Fehlfunktion des Körpers mittels eines Arzneimittel dessen pharmakologische Wirksamkeit im gastro-intestinalen Trakt abgeschwächt wird oder verloren geht, wie beispielsweise die Behandlung von Diabetes durch Insulin verwendet werden.

Eine Vorrichtung besteht aus einem oder mehreren Bauteilen und dient der Ausführung eines bestimmten medizinischen Zwecks insbesondere der Injektion eines Stoffes in den menschlichen oder tierischen Körper. Ein Bauteil besteht aus einer oder mehr als einer Komponente und dient der Erfüllung einer technischen oder nicht technischen Funktion. Eine Funktion ist technisch, wenn dabei eine Übertragung von Kraft, Arbeit, Energie, Material (Stoff), Daten und/oder Signalen, die Aufrechterhaltung der Struktur und/oder Form oder die Lagerung eines Stoffes bzw. Speicherung von Informationen betroffen ist. Eine Funktion ist nicht technisch, wenn die Eingabe oder Ausgabe von Information von oder an den Benutzer der Vorrichtung oder eines Stoffes von oder an den Benutzer der Vorrichtung betroffen ist.

Ein Bauteil kann beispielsweise Teil eines technischen Geräts sein, welches eine Teilfunktion im Verhältnis zur Gesamtfunktion des Geräts zur Verfügung stellt. Ein Bauteil ist beispielsweise ein Vorratsbehälter. Vorratsbehälter kann eine auswechselbare Ampulle enthaltend einen Stoff (insbesondere ein Medikament wie z. B. Insulin) sein. Die auswechselbare Ampulle kann sich insbesondere zur Verwendung in einem Insulinpens oder einer anderen Vorrichtung zur Injektion eines Medikaments in den menschlichen oder tierischen Körper eignen. Ein anderes Beispiel für ein technisches Bauteil ist eine Vorrichtung zum Pumpen oder eine Pumpe. Weitere Beispiele fürtechnische Bauteile sind insbesondere Spritzen, Nadeln, Kolbenstangen, Dosiereinrichtungen, mechanische Anzeigen, Schläuche, Dichtungen, Batterien, Motoren, Getriebe, elektronische Anzeigen, elektronische Speicher oder elektronische Steuerungen. Als Zweck im Zusammenhang mit der technischen Vorrichtung soll insbesondere die Bewegung von Flüssigkeitvon einem Ort zu einem anderen verstanden werden. Ein Zweck ist beispielsweise durch Bewegung eines Flüssigkeitsvolumens von einem Vorratsbehälter zu einer Ableitung definiert. Zweck kann auch die Injektion eines Medikaments in den menschlichen oder tierischen Körper sein.

Ein Bauteil kann mit einem oder mehreren anderen Bauteilen in technischer Weise verbunden sein, um gemeinsam einen Zweck zu erfüllen. Einetechnische Verbindung ist beispielsweise eine Verbindung von Bauteilen, die sich zur Übertragung von Kraft, Arbeit, Energie, Material (Stoff), Daten und/oder Signalen eignet. Verbunden werden können die Bauteile z. B. über eine mechanische Kupplung, eine feste mechanische Verbindung (Kleben, Schrauben, Nieten, Gestänge oder ähnliches), ein Zahnrad, eine Klinke, eine Sperre, einen metallischen Draht, ein Lichtleiter, eine Funkverbindung, ein elektromagnetisches Feld, einen Lichtstrahl oder ähnliches.

Injektion ist die Einbringung von Stoffen insbesondere von Flüssigkeiten mittels einer Kanüle samt Spritze oder funktionell vergleichbarer Vorrichtung wie insbesondere einen Pen in den menschlichen oder tierischen Körper. Man kennt unter anderem subkutane, intramuskuläre, intravenöse, intrakutane und intraartikuläre Injektion. Die subkutane Injektion erfolgt unter die Haut, sie ist relativ einfach auszuführen, wenig schmerzhaft und kann vom Patienten selbst vorgenommen werden. Die intramuskuläre Injektion erfolgt in den Muskel. Da hierbei größere Risiken bestehen, wie beispielsweise die schmerzhafte Verletzung von Knochenhaut, wird diese meist von medizinischem Personal vorgenommen. Die intravenöse Injektion erfolgt nach Venenpunktion direkt über eine Vene.
Bei der intrakutanen Injektion wird ein Arzneimittel direkt unter die Lederhaut verbracht. Bei der intraartikulären Injektion wird eine Flüssigkeit in ein Gelenk injiziert. Die Injektion eines Stoffes in den menschlichen oder tierischen Körper ist insbesondere zu unterscheiden von der Einbringung eines Stoffes durch eine Medikamentenpumpe, eine Infusion oder eine andere Art der kontinuierlichen und über einen gewissen Zeitraum erfolgenden Zuführung.

Eine Kanüle ist im Wesentlichen eine hohle Nadel, die gewöhnlich aus Metall (z. B. Stahl, Edelstahl, Gold, Silber, Platin) gefertigt ist. Das Ende der Kanüle ist häufig mit einem schrägen Schliff geschärft. Die Kanüle kann an einem Ende spitz und/oder geschärft und am anderen Ende stumpf sein, sie kann aber auch an beiden Enden spitz und/oder geschärft sein. Die Kanüle trägt an einem der beiden Enden einen meist konusförmigen Aufsatz aus beispielsweise Kunststoff mittels dessen ein Anbringen der hohlen Nadel zum Beispiel durch Aufstecken oder Aufschrauben an einem medizinischen Gerät wie beispielsweise einer Spritze, einem Medikamentenpen insbesondere einem Insulinpen, einem Medikamentenbehälter oder einer Medikamentenpumpe möglich ist. Die Kanüle dient in derfunktionellen Interaktion mit einer Spritze, einem Pen, einer Pumpe oder einem anderen dafür geeigneten medizinischen Gerät der Entnahme oder Zufuhr einer Flüssigkeit aus oder in den menschlichen oder tierischen Körper.

Die Angabe des Kanülendurchmessers (Auβendurchmesser) erfolgt meist in mm oder in Gauge (18 Gauge = 1,2 mm; 20 Gauge = 0,9 mm; 21 Gauge = 0,8 mm; 22 Gauge = 0,7 mm; 23 Gauge = 0,6 mm; 25 Gauge = 0,5 mm; 27 Gauge = 0,4 mm). Eine andere Kenngröße zur Charakterisierung der Kanüle ist deren Länge. Typische Längen von Kanülen sind 40 mm, 30 mm, 25 mm, 8 mm, 6 mm und andere Längen.

Ein medizinisches Gerät ist insbesondere ein Gerät zur Injektion des Stoffes in den menschlichen oder tierischen Körper. Neben einer Spritze kann ein solches Gerät zur Injektion ein Medikamentenpen wie beispielsweise ein Insulinpensein. Medikamentenpens sind in unterschiedlicher Form und für unterschiedliche Zwecke geeignet und von verschiedenen Herstellern auf dem Markt erhältlich (z. B. Optiklick, Optipen, Optiset).

Jeder Insulinpen muss zahlreichen Anforderungen hinsichtlich der Bedienfreundlichkeit genügen, um die sichere und fehlerfreie Anwendung zu ermöglichen. Grundvorrausetzung ist das Anzeigen der vorgewählten Dosis bzw. der Restmenge in der Ampulle. Weiterhin ist die Dosiseinstellung sowie der Abschluss des Injektionsvorgangs hörbar, fühlbar und sichtbar zu gestalten. Diese Sicherheitsanforderung ergibt sich vor allem aus den eingeschränkten Wahrnehmungsmöglichkeiten bei älteren Diabetes Typ-2 Patienten.

Neben nadelbehafteten Insulinpens kommen auch nadelfreie Injektionssysteme bei der Insulintherapie zum Einsatz. Ein aktuelles Anwendungsbeispiel für nadelfreie Injektionssysteme ist das Injektionssystem Injex der Fa. Rösch AG. Bei diesem Injektorwird das Insulin mit extrem hohem Druck durch eine Mikronadel in die Fettschicht der Haut geschossen. Eine vor der Injektion manuell gespannte Sprungfeder speichert die dafür notwendige Injektionsenergie. Das Injektat wird dabei homogen und konusförmig im Fettgewebe verteilt.

Ein nicht zu vernachlässigender Vorteil dieser Geräte ist die nadelfreie Injektion des Medikaments, die bei einigen Patienten die psychologische Hemmschwelle der Insulinapplikation herabsetzt. Fernerschließt die nadelfreie Injektion eine Infektion der Einstichstelle aus. Nachteilig gegenüber herkömmlichen Insulinpens erweist sich das Umfüllen des Insulins in spezielle Ampullen, die vergleichbar größere Masse des Geräts sowie das Mitführen weiteren Zubehörs zum Spannen der Feder.

Im Unterschied zu Insulinspritzen sind Insulinpumpen vollautomatische Infusionssysteme zur kontinuierlichen subkutanen Insulininjektion. Sie haben etwa die Größe einer Zigarettenschachtel und werden permanent am Körper getragen. Das kurzwirkende Insulin wird uber einen Katheder und einer in der Haut liegenden Nadel nach dem vom Patienten vorgegebenen Programm in das Hautgewebe gespritzt. Die Aufgabe der Insulinpumpe ist es, den kontinuierlichen Insulinausstoß der Bauchspeicheldrüse zur Senkung des Blutzuckerspiegels zu imitieren, ohne abereinen geschlossenen Regelkreis der Blutzuckerregulierung realisieren zu können. Aufgrund der kontinuierlichen und anpassbaren Zuführung des Insulins bieten diese Pumpen Vorteile vor allem für sportlich aktive Menschen bzw. solche mit stark wechselnden Tagesabläufen. Mit der Insulinpumpentherapie können starke Schwankungen des Blutzuckers, z. B. bei Diabetikern mit ausgeprägtem DAWN-Phänomen, ausgeglichen werden, die mit herkömmlichen Methoden nur mit erhöhtem Aufwand beherrschbar sind. Ein Nachteil ist, dass bei unterbrochener Insulinzufuhr aufgrund des fehlenden Insulinvorrats im menschlichen Körper schwere Stoffwechselentgleisung auftreten können. Insulinpumpen gibt es in verschiedenen technischen Ausführungen, wobei sich Geräte mit spritzeriartigen Behältern im Laufe der technischen Entwicklung durchgesetzt haben: Analog zu den Insulinpens mit Nadeln befindet sich das Insulin in einem Vorratsbehälter mit beweglichem Stopfen. Dieser wird durch eine motorisch angetriebene Kolbenstange bewegt.

Aufgrund der vollautomatischen und kontinuierlichen Insulinabgabe sind die Pumpen mit einer Vielzahl von Sicherungssystemen versehen, um den Anwender vor folgenschweren Fehlfunktionen zu schützen. Dies befreit aber nicht von einer eigenverantwortlichen und vorrausschauenden Nutzung des Gerätes.

Auf Basis der heutigen Injektionsgeräte und der technologischen Weiterentwicklung in der Medizin- und Mirkosystemtechnik ist ein Trend zu vollautomatischen miniaturisierten Medikamentendosierungssystemen zu verzeichnen. Die weitere Entwicklung könnte in Richtung implantierbarer und extrakorporaler Medikamentendosiersysteme verlaufen. Mit implantierbaren Insulinpumpen verfolgt man das Ziel, den Diabetiker vom täglichen Spritzen des Insulins zu befreien, ohne dabei ein externes Gerät am Körper tragen zu müssen.

Insulinpens sind in den wesentlichen ergonomischen und sicherheitstechnischen Merkmalen in der Norm ENISO 11608 konzentrieren. Diese schließt ebenfalls die geometrisch-stofflichen Eigenschaften der Insulinampullen und Pennadeln ein. Somit ist für den Benutzer die Handhabung und die Bedienung eines Pens weitgehend einheitlich und vom Modell unabhängig.

Auf den Inhalt der Norm EN ISO 11608, soweit sich dieser auf Insulinpens, Insulinampullen sowie Nadeln bezieht, wird hiermit ausdrücklich als Bestandteil vorliegender Offenbarung verwiesen.

In der konstruktiven Ausführung der Pens lassen sich zum Teil beachtliche Unterschiede bei den Pens der verschiedenen Hersteller feststellen. Diese sind beispielsweise mit der Bestimmung für unterschiedliche Zielgruppen (Kinder, ältere Menschen) begründet. Aufgrund der Anforderungen aus der Norm EN ISO 11608 beschränken sich die Unterschiede vor allem auf den Injektionsmechanismus und den Auslösemechanismus. Der Dosiswähler und die Dosieranzeige unterliegen meist ergonomischen Anforderungen und ergeben sich aus den konstruktiven Allgemeinbedingungen des jeweiligen Modells.
Das wesentliche Funktionselement eines Insulinpens ist der Injektionsmechanismus. Er bestimmt die Bauform und Baugröße des Pens sowie die konstruktive Ausführung des Auslösemechanismus und des Dosiswählers. Der Mechanismus übersetzt die am Dosiswähler voreingestellte Dosis mit der vom Auslösemechanismus kommenden Injektionsenergie in einen Injektionshub des Stopfens in der Ampulle. Diese Energie wird entweder direkt auf den Injektionsmechanismus oder durch bewegungsumformende Getriebe übertragen.

Der Injektionsmechanismus in Gestalt der Kolbenstange ist technisch in vielfältiger Form realisierbar. Bei derzeitig auf dem Markt verfügbaren Insulinpens haben sich Lösungen mit einer starren (z. B. Gewindespindel, Zahnstange) oder einer flexiblen (z. B. gebogenen Zahnstange, gebogenen Druckfeder) Bauform etabliert. Andere mögliche Ausführungen wie teleskopischer Kolbenstange (z. B. Schraubengetriebe, Zugmittelgetriebe, Druckmittelgetriebe, Koppelgetriebe) kommen bei den gegenwärtig im Handel verfügbaren Insulinpens nicht zum Einsatz.

Die konstruktiven Lösungen der starren und flexiblen Bauform sind sehr unterschiedlich und vom Typ des Pens, d. h. wieder verwendbarer Pen oder Fertigpen, abhängig. Als Kolbenstangen kommen Gewindespindeln oder Zahnstangen bzw. Kombinationen beider zum Einsatz. Beim Dosierwähler wird mit Hilfe von Rastvorrichtungen ein der Dosis entsprechender Drehwinkel voreingestellt und mit nachgeschalteten Schrauben- und Zahnradgetrieben auf den Injektionsmechanismus übertragen sowie in den Injektionshub transformiert.

Die Abgabe des Medikaments erfolgt durch Vorgabe eines Injektionshubs und der resultierenden Verschiebung des Stopfens. Die abgegebene Flüssigkeitsmenge ist vom Injektionshub und dem Innendurchmesser der Ampulle abhängig. Zur Vermeidung von Dosierfehlern sind entsprechend Herstellervorgaben und der Norm EN ISO 11608 Luftblasen vollständig zu entfernen. Weiterhin ist nach Abgabe der Flüssigkeit eine hinreichend lange Wartezeit einzuhalten, um einen stationären Zustand, d. h. Normaldruck der Flüssigkeit und Relaxation des Stopfens in der Ampulle zu gewährleisten.

Der Vorratsbehälter für das Medikament (auch als Ampulle oder Kartusche bezeichnet) beeinflusst den Aufbau und die Funktionsstruktur des Medikamentenstifts. Man kann hierbei als Teilfunktionen unterschieden einmal eine Schutzfunktion für das Medikament, dann eine Förderfunktion und schließlich eine Kopplungsfunktion zum Injektionssystem des Medikamentenstiftes. Die Schutzfunktion wird durch die Ampulle insgesamt, d. h. durch Stopfen, Glaskörper und Dichtscheibe realisiert. Die Förderfunktion für das Medikament vermittelt der Stopfen, welcher mit Hilfe des Injektionsmechanismus verschoben wird und eine Volumenänderung in der Ampulle bewirkt. Die Kopplungsfunktion zum Injektionssystem schließlich wird durch Dichtmittel (z. B. Dichtscheibe) hergestellt.

Bei einem automatischen Medikamentenstift (z. B. automatischer Insulinstift oder Insulinpen) wird die Injektionsenergie von einem Antrieb mit nachgeordnetem Getriebe aufgebracht. Zusätzlich sind Energieversorgung und Steuereinrichtung notwendig.

In einem nicht-erfindungsgemäßen Injektionsmechanismus erfolgt die Förderung des Medikaments (z. B. durch Insulin) nicht überdie Verschiebung des Stopfens mittels eines Injektionsmechanismus sondern über die Einführung einer Pumpvorrichtung. Die Pumpvorrichtung wird zwischen Ampulle und Injektionssystem eingefügt und ist mit entsprechenden Schnittstellen zu versehen.
Die Pumpvorrichtung kann mit einer Flusssensorik versehen werden. Sie steht in direktem Kontakt zum Medikament z. B. Insulin, woraus sich zusätzliche Anforderungen wie verminderte Keimzahl, Sterilität, Biskompatibilität u. a. ergeben können.
Bei Anwendung dieses Funktionsprinzips ändern sich im Vergleich zu einem herkömmlichen Medikamentenstift zur Injektion (z. B. einen Insulinpen) zahlreiche Zustandsgrößen z. B. der (Flüssigkeitsdruck im Medikamentenbehälter) da beim Heraussaugen des Medikaments ein Unterdruck entsteht.

Insulinampullen dienen als Primärpackmittel für das Medikament und müssen hohe Standards erfüllen. Dies betrifft die MaßhaltigkeitderAmpulle im Hinblick auf die Dosiergenauigkeit und Kompatibilität mit anderen Komponenten. Die Norm EN ISO 11608-3 greift diese Forderungen auf und beschreibt die grundlegenden Gesichtspunkte und den geometrisch-stofflichen Aufbau, ohne die Ampullengestaltung unnötig einzuschränken. Ebenso muss die pharmazeutische Dichtigkeit der Ampulle gewährleistet sein.

Die Ampullen bestehen aus mehreren Teilkomponenten. Die wichtigste ist der Zylinder aus pharmazeutischem Glas mit einer hohen Neutralität u nd chemischen Beständigkeit gegenüber dem Insulin. Vor der Befüllung wird die Oberflächenqualität des Zylinders durch Silikonisierung verbessert. Diese Oberflächenvergütung verringert die Gleit- und Losbrechkräfte des Stopfens, erhöht die Dosiergenauigkeit und verringert das Herauslösen von Glasbestandteilen bei langer Lagerzeit. Der Grad der Silikonisierung korreliert dabei mit der Höhe der Reibkräfte des Stopfens, wobei ein Grenzwert durch die Sensibilität des Insulins gegenüber dem Silikon gesetzt wird.

Die Ampulle wird beiderseitig durch elastomere Verschlussteile, den Stopfen und die Dichtscheibe, abgedichtet. Entscheidend sind dabei die nachgewiesene mechanische Dichtigkeit bei verschiedenen Drucksituationen sowie die mikrobiologische Dichtigkeit gegenüber Keimen in Langzeitversuchen. Wichtig sind weiterhin die maximal vertretbaren Stopfenkräfte und die Anzahl von Durchstichen der Dichtscheibe mit einer Kanüle.

Pennadeln sind sterile Einwegprodukte, die zum Einsatz kommen, um das Insulin aus der Ampulle in das Zielgewebe zu leiten. Sie unterliegen ebenso wie Ampullen strengen Anforderungen, da erst durch das Zusammenspiel beider Komponenten die eigentliche Funktionalität des Insulinpens erzielt wird. Die Nadel besteht aus einer beiderseitig geschliffenen Kanüle, die in einem Ampullenansatzstück eingefasst ist. Optimierte Kanülenschliffe ermöglichen dem Patienten ein weitgehend schmerzfreies Einstechen in das Zielgewebe und verursachen beim Wiederrherausziehen nur geringe Gewebeschädigungen. Ebenso wird die Ampullendichtscheibe ohne starke Fragmentation durchbohrt. Dies ist eine zwingende Vorraussetzung, da auch bei regelmäßigem Wechsel der Nadel die Dichtigkeit der Ampulle gewährleistet werden muss. Das Ampullenansatzstück sorgt für einen festen Sitz auf dem Insulinpen.

Auch wenn Pennadeln nach zwei- bzw. mehrmaligem Gebrauch für das Auge kaum sichtbare Abnutzungserscheinungen aufweisen, sollten sie dennoch nach jeder Injektion aus Gründen der Sterilität gewechselt werden. Zudem kann auskristallisiertes Insulin die Nadel verstopfen. Fernerhin gelangt bei Temperaturschwankungen Luft in die Ampulle, die gleichermaßen Dosierfehler verursacht. So bewirkt bereits ein Temperaturwechsel von 15 K, dass bis zu 15 µl Luft in die Ampulle eintreten.

Die Mikrofluidik ist ein Teilgebiet der Mikrosystemtechnik und umfasst Entwurf, Herstellung, Anwendung und Untersuchung von Mikrosystemen, die Fluidmengen in Kanalquerschnitten mit Abmessungen von 1 µm bis 1 mm manipulieren und behandeln. Mikrofluidische Systeme kommen in der Medizintechnik, der Biochemie, derchemischen Verfahrenstechnik und Analytik sowie der Mirkoreaktionstechnik zum Einsatz. Diese Mikrosysteme können dabei Abmessungen im Millimeter- und Zentimeterbereich haben, da für praktische Anwendung die Fluidmenge und nicht die Größe des mikrofluidischen Systems von Bedeutung ist. Zudem weisen solche Systeme aufgrund geringer Fluidmengen und oftmals kleiner Systemgrößen bedeutende Unterschiede gegenüber konventionellen fluidischen Systemen auf. Mit der Miniaturisierung ändert sich das Verhalten der Fluidströmung, da oberflächengebundene Effekte sowie elektrostatische und elektrokinetische Kräfte dominieren. daher sind neue Herangehensweisen für Entwurf, Herstellung und Charakterisierung von mikrofluidischen Komponenten, z. B. Mikropumpen und Sensoren, notwendig. Infolge konstanter Energiedichte der Aktuatoren sinkt deren Abgabeleistung, sodass diese nicht mit der von herkömmlichen Komponenten im Makrobereich vergleichbarsind. Aus diesem Grund werden häufig externe Aktuatoren eingesetzt, welche die Abmessungen des Gesamtsystems mitunter erheblich vergrößern. Weiterhin begrenzen Physik und Chemie der zu transportierenden Teilchen und Moleküle die Miniaturisierung mikrofluidischer Komponenten.

Diabetes mellitus ist eine Erkrankung, bei der der Körper selbst keine oder nicht mehr ausreichende Mengen an Insulin produzieren bzw. adäquat verwenden kann. Insulin wird benötigt, um Zucker aus dem Blut in die Körperzellen zu transportieren. Dar Blutzuckerspiegel wird ständig in engen Grenzen konstant gehalte (60-100 mg % bzw. 3,33-5,55 mmol/l). Dies erfolgt durch das Zusammenspiel der beiden Hormone Insulin und Glucagon.

Die Diagnose von Diabetes mellitus erfolgt nach Blutentnahme mittels entsprechender Laborgeräte. Es muss mindestens zweimal zu unterschiedlichen Zeitpunkten ein erhöhter Blutzuckerwert nachgewiesen werden, um die Diagnose zu erhärten.

Von Diabetes mellitus spricht man, wenn der Glukosewert gemessen im Blutplasma in wenigstens einem der angeführten Fälle den angegebenen Wertübersteigt:
a) Nüchtemblutzucker - 7,0 mmol/l, bzw. 126 mg/dl
b) Blutzucker zwei Stunden nach Gabe von 75 mg Glukose (oraler Glukose-Toleranztest) -11,1 mmol/l bzw. 200 mg/dl
c) Blutzucker 11,1 mmol/l bzw. 200 mg/dl verbunden mit starkem Durst (Polydipsie), häufigem Wasserlassen (Polyurie) oder Gewichtsverlust.

Unbehandelt führt Diabetes zu erhöhten Blutzuckerwerten, die zu verschiedenen Symptomen und Spätfolgen führen können wie beispielsweise Polyneuropathie, Mikroangiopathie, Makroangiopathie, Retinopathie, Nephropathie und anderen. Das Risiko von diabetischen Spätschäden ist umso geringer, je niedriger die nichtenzymatische Glykierung der Erythrozyten (HbA1c-Wert) ist

Diabetisches Koma ist eine lebensgefährliche Akutkomplikationvon Diabetes. DerBlutzuckerwertkann dabei über 1000 mg/dl erreichen einhergehend mit einer starken Übersäuerung des Blutes (metabolische Azidose). Diabetisches Koma kann unter anderem ausgelöst werden durch Infekte, Aufnahme von zu viel Kohlehydraten, Alkoholmissbrauch oder falsche Dosierung des Insulin.

Man unterscheidet den Typ 1 Diabetes von Typ 2 Diabetes. Beim Typ 1 Diabetes liegt von Anfang an ein absoluter Insulinmangel vor, der nur mit Insulingabe behandelt werden kann.
Der Typ 2 Diabetes ist gekennzeichnet durch eine verminderte Insulinempfindlichkeit und einen relativen Insulinmangel. Typ 2 Diabetes lässt sich meist zunächst mit diätetischen Maßnahmen und Tabletten behandeln. Häufig wird im Verlauf der Erkrankung eine Insulin-Substitution nötig.

Der Typ 2 Diabetes ist vorwiegend in den industrialisierten Ländern eine weit verbreitete Krankheit geworden. Als Hauptursache gelten Überernährung, Bewegungsmangel und Übergewicht. Dem Typ 2 Diabetes lässt sich durch Bewegungs-Training und diabetische Maßnahmen, insbesondere auf Gewichtsreduktion abzielend, wirksam entgegenwirken. Daneben können im Falle des Typ 2 Diabetes orale Antidiabetika wie z. B. Acarbose, Biguanide, Sulfonylharnstoff, Glitazon und andere eingesetzt werden. Die Therapie, unter Verwendung von Insulin wird erforderlich, wenn mittels der genannten Maßnahmen der Blutzuckerspiegel nicht mehr mit ausreichender Nachhaltigkeit im oder nahe dem Normbereich gehalten werden kann.

Zur Insülintherapie stehen verschiedene Insuline zur Verfügung. Man unterscheidet gewöhnlich nach Wirkdauer oder chemischer Struktur. Ein Analoginsulin weist im Vergleich zu Humaninsulin an einzelnen Positionen unterschiedliche Aminosäuren auf. Dadurch können sich die Eigenschaften ändern.

Zu den schnellwirksamen Insulinen rechnet man das Humaninsulin sowie verschiedene schnell und kurz wirksame Insulin-Analoga wie Glulisin (Handelsname: Apidra), Lispro (Handelsname: Humalog) undAspart (Handelsname: Novo Rapid).

Langsam wirkende oder Verzögerungsinsuline sind das NPH-Insulin (durch Neutral Protamin Hagedorn verzögert wirkendes Humaninsulin), Zinkinsuline und verschiedenen Insulin-Analoga wie Glargin (Handelsname: Lantus) und Detemir (Handelsname: Levemir).

In der Insulintherapie werden außerdem Mischinsuline und neuerdings inhalative Insuline verwendet.

Mischinsuline bestehen aus einem schnellwirksamen Insulin und einem Verzögerungsinsulin in verschiedenen Mischungsverhältnissen. Üblich sind Mischungen von 10/90 %, 25/75 %, 30/70 %, 50/50 %. Eine Insulintherapie muss immer von regelmäßigen Bestimmungen des Blutzuckerspiegels begleitet werden.

Bei der konventionellen Insulintherapie wird zu festgesetzten Zeiten eine bestimmte Menge Mischinsulin gespritzt. Die intensivierte konventionelle Insulintherapie kommt überwiegend bei der Therapie von Typ-1-Diabetikern zum Einsatz. Hierbei wird eine Grundversorgung über ein Verzögerungsinsulin (Basis) gewährleistet und zusätzlich zu den Mahlzeiten ein schnellwirksames Insulin (Bolus) gegeben.

Die kontinuierliche subkutane Insulininfusion mittels einer Pumpe kommt überwiegend für Typ-1-Diabetiker in Frage. Das Insulin wird nicht gespritzt sondern von einer kleinen Pumpe in den Körper geleitet. Die Pumpe befindet sich dauernd am Körper. Das Insulin wird über einen Katheter mit Kanüle zugeführt. Die Insulinpumpe gibt gewöhnlich schnell wirkendes Insulin in kleinen gleichmäßigen Abständen über einen längeren Zeitraum ab.

Glucagon-like-Reptid 1 (GLP1) zählt neben Glucose-dependant insulinotropic peptid (GIP) zu den wichtigsten Vertretern der Inkretine. Inkretine werden als Hormone im Darm gebildet und regulieren unter anderem den Blutzuckerspiegel durch Anregung der Insulin-Ausschüttung im Pankreas.

Die Menge der gebildeten Darmhormone ist abhängig von der oral aufgenommenen Menge an Kohlenhydraten. Der Spiegel an GLP1 steigt nach oraler Glukose-Aufnahme sehr viel stärker als nach der intravenösen Gabe von Glukose. Mit Untersuchungen konnte gezeigt werden, dass die intravenöse Infusion und die subkutane Injektion von GLP1 bei Typ 2-Diabetikern in vielen Fällen zu einer kompletten Normalisierung des Blutzukkerspiegels führt. Problematisch ist, dass GLP1 innerhalb sehr kurzer Zeit durch Dipeptidylpeptidase IV (DPP-IV) inhibiert wird. Eine subkutane Injektion von GLP1 kann nur über ca. 1-2 Stunden wirksame Plasmakonzentrationen aufrechterhalten. Eine Lösung in Richtung nachhaltiger Wirkung von GLP1 könnte bei der Entwicklung länger wirksamer GLP-Analoga oder auch der Hemmung von DPP-IV durch Arzneimittel zu finden sein.

Wachstumshormone sind Substanzen, die bei Menschen, Tieren und Pflanzen das Wachstum stimulieren. Man kennt beispielsweise das Somatotropin (Mensch), das bovine Somatotropin (Rind) und Auxin sowie Gibberellsäure (Pflanze).

Somatotropin (STH) ist auch unter den Bezeichnungen Human Growth Hormone (HGH), Growth Hormone (GH) oder Wachstumshormon (WH) bekannt. STH ist ein Peptidhormon mit 191 Aminosäuren. Die Bildung erfolgt im Hypophysenvorderlappen unter Regulierung des Somatotropin-releasing-Faktor (SRF; GHRH; GRF) aus dem Hypothalamus. STH ist für ein normales Längenwachstum unbedingt erforderlich. Bei verminderter Produktion oder einem verminderten Ansprechen der Zellen auf STH kommt es zu einem Kleinwuchs. Bei einer Überproduktion kommt es zu Riesenwuchs bzw. Akromegalie.

Kleinwuchs, der durch Mangel am Wachstumshormon verursacht ist, wird seit einigen Jahren durch Gabe von STH behandelt. Die Gewinnung erfolgte zuerst aus Hypophysen von Toten, bevor das STH seit 1985 gentechnologisch hergestellt werden konnte.

Interferone werden als Gewebehormone von menschlichen oder tierischen Leukozyten, Fibroblasten oder T-Lymphozyten gebildet. Ein Interferon ist ein Protein oder Glykoprotein mit einer immunstimulierenden (z. B. antiviral) oder antihormonen Wirkung. Man unterteilt die Interferone in Alpha-Interferone, Beta-Interferone und Gamma-Interferone. Interferone sind von verschiedenen Herstellern gegen Indikationen wie Viruserkrankungen (z. B. SARS), Krebs, multiple Sklerose, Hepatitis B/C, Hepatitis C erhältlich.

Bei einem Impfstoff handelt es sich um eine biologisch oder gentechnisch hergestellte Zusammensetzung enthaltend unter anderem einzelne Proteine und/ oder RNA bzw. DNA Bruchstücken und/oder abgetötete oder abgeschwächte Erreger (z. B. Influenza, SARS, Pockervirus, Erreger von Masern, Mumps, Röteln, Kinderlähmung, Erreger des Keuchhustens).

Man kennt Lebendimpfstoffe (z. B. Kuhpokken), attenuierte Lebendimpfstoffe mit abgeschwächten Viren oder Bakterien (z. B. MMR-Impfstoff; Gelbfieber, Kinderlähmung) und Totimpfstoffe mit inaktivierten oder abgetöteten Viren oder Bakterien bzw. deren Bestandteilen (z. B. Influenza, Cholera, Beulenpest, Hepatitis A).

Heparine sind therapeutisch eingesetzte Substanzen zur Hemmung der Blutgerinnung. Heparine bestehen aus jeweils abwechselnden Folgen von D-Glucosamin und D-Glucuronsäure bzw. L-Iduronsäure. Kettenlängen bestehend aus 5 Einheiten können bereits gerinnungshemmend sein.

Die Polysaccharidketten haben meist ein Molekülgewicht zwischen 4000 und 40000. Neben unfraktionierten Heparinen kommen auch niedermolekulare fraktionierte Heparine mit einem Molekulargewicht von ca. 5000 zum Einsatz. Heparine werden nicht aus dem Magen-Darm-Trakt resorbiert, sonder müssen parenteral appliziertwerden. Heparine wirken überdie Bindung an Antitrhombin III und eine dadurch beschleunigte Inaktivierung von aktivierten Gerinnungsfaktoren.

Lovenox (auch bekannt als Clexane) ist eine kommerziell erhältliche Arzneimittelzubereitung mit dem pharmakologisch aktiven Wirkstoff Enoxaprin-Natrium. Der Wirkstoff zählt zu den niedermolekularen Heparinen mit einer linearen Dosis-Wirkungs-Beziehung und einer konstant hohen Bioverfügbarkeit.

Indikationsgebiete von Lovenox sind die Primärprohylaxe tiefer Venenthrombosen, die Therapie tiefer Venenthrombosen mit und ohne Lungenembolie, die Therapie von instabiler Angina pectoris und des so genannten Nicht-Q-Wellen Herzinfarkts sowie der Thromboseprophylaxe und Gerinnungshemmung während der Hämodialyse.

### Beispiel

Aufbau eines Messapparaturzum Durchleuchten einer Ampulle, Aufnehmen des Schattenbildes, Übertragen der Messwerte auf einen PC und anschließender Bilderkennung.

Der Messaufbau besteht aus einer Lichtquelle, Spaltblenden, einer Ampulle mit Einspannvorrichtung und einem Zeilensensor.

Die Lichtquelle beinhaltet jeweils alternativ eine diffus strahlende LED Zeile, eine LED-Zeile aus LED's mit geringem Öffnungswinkel oder Punktquelle mit Sammellinse. Als Zeilensensorwird eine CCD-Zeilenkamera ohne Objektiv mit Wellenlängen abhängiger Empfindlichkeit (Maximum im roten Spektralbereich) verwendet.

Zur Verringerung der Streustrahlung befinden sich vor und hinter der Ampulle Spaltblenden.

Die Ampulle wird in einer Ebene in Mittellage durchleuchtet. Das Schattenbild besteht aus Halbschatten und Kernschatten des Stopfens. Nach Übertragung der digitalisierten Intensitätsmesswerte der einzelnen Sensorelemente zum PC wird durch Bestimmen der Pixel, die eine bestimmte Helligkeit unterschreiten (Schwellwertvergleich) die Stopfenposition durch eine entsprechende Software ermittelt.

Das Erkennungssystem eignet sich insbesondere in Injektionspens für Maßnahmen zur Dosierung bzw. Bestimmen der Applikationsmenge.

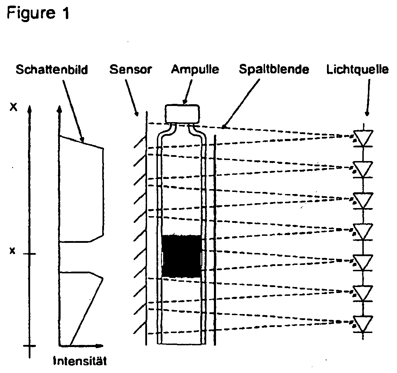

Die Messapparatur besteht aus den drei Hauptkomponenten Lichtquelle, Zeilensensor mit Blende und Auswerteelektronik (Figur 1). Als Lichtquelle werden in Reihe angeordnete Leuchtdioden mit einem Öffnungswinkel von etwa 6° verwendet. So gelingt bei geeigneter Anordnung eine gleichmäßige Ausleuchtung der Ampulle zu erzielen, die für die Kantenerkennung notwendig ist. Zwischen der Lichtquelle und der Ampulle befindet sich eine Spaltblende zur Verbesserung des Kontrastes und der Messgenauigkeit. Sie ist notwendig, um Helligkeitsschwankungen am Sensor durch unterschiedliche Brechung des Lichtes bei gefüllter und leerer Ampulle auszugleichen bzw. um Streulicht durch Totalreflexion am Glaszylinder zu vermeiden.

Der Zeilensensor hat insgesamt 1280 Pixel mit einem Pixelabstand von 63,5 µm und erfasst das Schattenbild der Ampulle vollständig. Die lichtempfindlichen Pixel des Sensors wandeln das einfallende Licht in elektrische Signale um, deren Werte von der Lichtintensität und der Integrationszeit abhängig sind. Die Sensorelektronik übergibt Sensorwerte mit einer Abstufung von 8 Bit an eine Schnittstelle zur Auswerteelektronik. Dunkle Bereiche, z.B. im Kernschatten hinter dem Stopfen, besitzen dabei einen Wert nahe Null. Bei Betrieb mit Umgebungslicht ist der Sensor nach etwa 2 ms übersteuert und die Auswertung des Schattenbildes nicht mehr möglich. Daher befindet sich das Sensorsystem bei dem Funktionsmuster abgeschirmt in einem lichtdichten Gehäuse. Die Auswertung der Sensordaten erfolgt mit einem Messrechner und der Software LabView. Ein angepasstes Programm verarbeitet die Sensordaten und berechnet mit einem Kantenerkennungsalgorithmus die Stopfenposition. Die Taktzeit für die Berechnung der Stopfenposition beträgt bedingt durch die Rechenleistung des Messrechners, der Datenübertragung zwischen Sensor und PC sowie der Intensität des Schattenbildes etwa 20 ms. Die Steuerung des Dosiervorganges bzw. der Fördervorrichtung erfolgt söftwareseitig durch das Umrechnen der Stopfenposition in eine volumetrische Größe. Ein charakteristisches Schattenbild mit den typischen optischen Effekten in Korrelation zur Stopfenposition zeigt Bild 1. Offensichtlich ist die bereits beschriebene unterschiedliche Helligkeitsverteilung zwischen gefülltem und leerem Glaszylinder. Zusätzlich sind auch die Störungen am Ampullenkopf und -ende bedingt durch die Ampullengeometrie gut zu erkennen. Dies erfordert eine Fallunterscheidung bei der Kantenerkennung und die Teilung der Ampulle in mehrere Bereiche. Die höchste Messgenauigkeit wird dabei im mittleren Bereich der Ampulle erzielt, da dort eine homogene Glasstrüktur vorhanden ist.

### Bild 1: Charakteristische Intensitätsverteilung an derGlasampulle bei parallelem Lichteinfall

An Ampullenschulter und-ende treten Reflexionen durch Krümmung bzw. Inhomogenität des Glases auf, die eine Schwellwerterkennung behindern. Die Erkennung der Stopfenposition erfolgt in drei Schritten, der Kompensation von Taktzeitabweichungen, der Kantenerkennung und der Berechnung der Stopfenposition bei Korrektur optischer Effekte. Im ersten Schritt werden Fehler des Intensitätswertes aufgrund von Taktzeitabweichungen rechnerisch kompensiert. Beim normalen Betrieb treten aufgrund des Einsatzes eines Messrechners Abweichungen in der Taktzeit bzw. in der Integrationszeit von bis zu 2 ms auf. Daraus ergeben sich auch bei gleicher Beleuchtung unterschiedliche Helligkeitswerte am jeweiligen Pixel, die durch das Messen dertatsächlichen Integrationszeit kompensiertwerden können. Weiter lässt sich die Auswirkung des Pixelrauschens durch Mittelwertbildung reduzieren. Im zweiten Schritt werden die Kanten mittels Schwellwerterkennung ermittelt. Die Helligkeitsverteilung in
Bild 1 zeigt eine deutliche Abtrennung zwischen dem Schattenbereich des Stopfens und dem hellen, direkt beleuchteten Bereich. Dabei ergibt sich die erste Kante aus der Position des Pixels, dessen Helligkeit als erster unter einem zuvor festgelegten Schwellwert liegt. Die zweite Position bestimmt sich aus der Pixelposition mit einem über dem Schwellwert liegenden Helligkeitswert. Liegt der Schwellwert zwischen zwei Pixeln, wird die Position durch Interpolation bestimmt, was zugleich die Auflösung der Kantenposition verbessert. Im dritten Schritt wird anhand der Stopfenkanten die Stopfenposition berechnet. Dazu muss abhängig von der Stopfenposition eine Fallunterscheidung vorgenommen werden:
1) Im Bereich 1 an der Startposition wird die Stopfenposition anhand der Vorderkantenposition berechnet. Der maximale Messfehler entspricht dem einfachen Pixelabstand von 64 µm.
2) Im Ampullenbereich 2 ergibt sich die Stopfenposition aus der Position der Vorder- und Hinterkante. Der maximale Messfehler beträgt in diesem Bereich etwa 32 µm, was einer halben Pixelbreite entspricht.
3) Im Ampullenbereich 3 wird die Position der Hinterkante des Stopfens genutzt. Daraus ergibt sich ein Messfehler einer einfachen Pixelbreite von etwa 64 µm. Für eine fehlerfreie Übergabe der Stopfenposition ist es möglich ebenfalls die Stopfenbreite heranzuziehen.

Beim Umschalten zwischen den einzelnen Bereichen wird die gemessene Stopfenbreite zu Hilfe genommen, um ein Springen der Stopfenposition zu vermeiden.

Beschreibung der Figuren:
Figur 1: Sensorsystem zur Erkennung der Stopfenposition (ohne Auswerteelektronik)

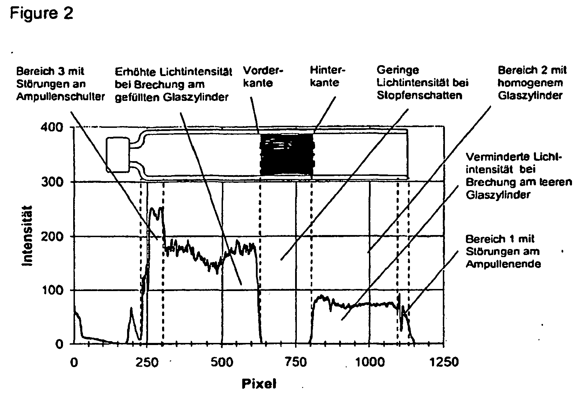

Figur 2: Charakteristische Intensitätsverteilung an der Glasampulle bei parallelem Lichteinfall

## Patentansprüche

1. Verfahren zur Bestimmung der Position eines Stopfens einer Medikamentenampulle in einem medizinischen Gerät entlang einer Wegstrecke mittels
a) einer Lichtquelle und
b) einer Halterung, welche den Stopfen längs einer Wegstrecke beweglich fixiert und
c) einer lichtempfindlichen Sensoroberfläche
**gekennzeichnet dadurch, dass** zuerst durch Bestrahlung des Stopfens mit Licht aus a) ein Schattenbild des Stopfens auf der Sensoroberfläche erzeugt wird, dann die Daten bezüglich des Schattenbildes von einer Datenverarbeitungseihheit in die Position des Stopfens entlang der Wegstrecke umgerechnet werden wobei abhängig von der Stopfenposition eine Fallunterscheidung vorgenommen wird:
- bei der in einem ersten Bereich an einer Startposition die Stopfenposition anhand der Vorderkantenposition berechnet wird,
- bei der in einem zweiten Bereich die Stopfenposition aus der Position der Vorder- und Hinterkante berechnet wird, und
- bei der in einem dritten Bereich die Stofenposition anhand der Hinterkantenposition berechnet wird.

2. Verfahren nach Anspruch 1, **gekennzeichnet dadurch, dass** die Position des Stopfens in einer Insulinampulle vorliegt.

3. Verfahren nach Anspruch 1 bis 2, **gekennzeichnet dadurch, dass** eine Datenverarbeitungseinheit im medizinischen Gerät integriert ist.

4. Verfahren nach Anspruch 1 bis 2, **gekennzeichnet dadurch**, das eine separate Datenverarbeitungseinheit zusammen mit dem medizinischen Gerät betrieben wird.

5. Verfahren nach einem oder mehreren der Ansprüche 1 bis 4, **gekennzeichnet dadurch, dass** zwischen a) und b) und/oder b) und c) eine Blende angebracht ist.

6. Verfahren nach einem oder mehreren der Ansprüche 1 bis 5, **gekennzeichnet dadurch, dass** die Lichtquelle aus einer LED-Zeile besteht.

7. Verfahren nach Anspruch 6, **gekennzeichnet dadurch, dass** die LED-Zeile diffus strahlt.

8. Verfahren nach Anspruch 6, **gekennzeichnet dadurch, dass** die einzelnen LED's der LED-Zeile einen geringen Öffnungswinkel aufweisen.

9. Verfahren nach Anspruch 6, **gekennzeichnet dadurch, dass** zwischen LED-Zeile und Stopfen eine Sammellinse eingefügt ist.

10. Verfahren nach Anspruch 9, wobei die Sammellinse eine Zylinderlinse ist.

11. Verfahren nach Anspruch 1 bis 10, **gekennzeichnet dadurch, dass** die Lichtquelle rotes Licht erzeugt.

12. Verfahren nach einem oder mehreren der Ansprüche 1 bis 5, **gekennzeichnet dadurch, dass** durch mindestens 2 nebeneinander ausgerichteten Lichtquellen Laserlicht erzeugt wird.

13. Verfahren nach Anspruch 12, **gekennzeichnet dadurch, dass** rotes Laserlicht erzeugt wird.

14. Verfahren nach einem oder mehreren der Ansprüche 1 bis 13, **gekennzeichnet dadurch, dass** die Sensoroberfläche aus einer Reihe angeordneter Sensorelemente besteht.

15. Verfahren nach Anspruch 14, **gekennzeichnet dadurch, dass** die Sensorelemente aus einer CCD-Zeilenkamera bestehen.

16. Verfahren nach Anspruch 14 oder 15, **gekennzeichnet dadurch, dass** die Empfindlichkeit bei rotem Licht am größten ist.

17. Vorrichtung zur Durchführung eines Verfahrens gemäß einem oder mehreren der Ansprüche 1 bis 16, diese Vorrichtung enthaltend mindestens
a) eine Lichtquelle; und
b) eine Halterung, welche einen Stopfen beweglich längs einer Wegstrecke fixiert; und
c) eine lichtempfindliche Sensoroberfläche und
d) eine Datenverarbeitungseinheit.

18. Verwendung einer Vorrichtung gemäß Anspruch 17 zum Zusammenbau eines medizinischen Geräts, welches zur Verabreichung eines Arzneimittels in den menschlichen oder tierischen Körper unter Umgehung des gastro-intestinalen Trakts geeignet ist.

19. Verwendung einer technischen Vorrichtung gemäß Anspruch 18, **gekennzeichnet dadurch, dass** das Arzneimittel Insulin ist.

20. Medizinisches Gerät zur Injektion eines Arzneimittels in den menschlichen oder tierischen Körper, das medizinische Gerät umfassend unter anderem
a) einen Grundkörper zur Montierung von mindestens einem technischen Bauteil;
b) ein technisches Bauteil in Gestalt eines Aufnahmebehälters für ein Arzneimittel;
c) ein technisches Bauteil in Gestalt eines Vorschubmechanismus;
d) ein technisches Bauteil in Gestalt einer Dosiervorrichtung;
e) ein technisches Bauteil in Gestalt einer Anzeige;
f) ein technisches Bauteil in Gestalt eines Auslösemechanismus zum Ingangsetzen und Durchführen der Injektion;
**gekennzeichnet dadurch, dass** zusätzlich mindestens eine Vorrichtung gemäß Anspruch 17 enthalten ist.

21. Medizinisches Gerät nach Anspruch 20, **gekennzeichnet dadurch, dass** es in Form und Funktion eines Insulinpen vorliegt.

22. Medizinisches Gerät nach Anspruch 20 oder 21, **gekennzeichnet dadurch, dass** mindestens ein Mittel zur Speicherung und/oder Verarbeitung von Daten und/oder Signalen sowie mindestens eine Schnittstelle zur Übertragung von Daten und/oder Signalen zu und/oder von einer externen technischen Einheit, welche zur Speicherung und/oder Verarbeitung von Daten und/oder Signalen ausgelegt ist, enthalten ist.

23. Medizinisches Gerät nach Anspruch 22, **gekennzeichnet dadurch, dass** die externe technische Einheit aus einem PC besteht, auf welchem ein Programm zur Speicherung und/oder Verarbeitung von Daten und/oder Signalen installiert ist.

24. Medizinisches Gerät gemäß einem oder mehreren der Ansprüche 20 bis 23, zur Injektion von Insulin.

25. Medizinisches Gerät gemäß Anspruch 24, worin das Insulin ein langwirksames und/oder ein kurzwirksames Insulin ist.

26. Medizinisches Gerät gemäß einem oder mehreren der Ansprüche 20 bis 23, zur Injektion von GLP-1.

27. Medizinisches Gerät gemäß einem oder mehreren der Ansprüche 20 bis 23, zur Injektion von Lovenox.

28. Herstellung eines medizinischen Geräts gemäß einem oder mehreren der Ansprüche 20 bis 27 zur Injektion eines Arzneimittels in den menschlichen oder tierischen Körper wobei
a) ein Grundkörper zur Montierung von mindestens einem technischen Bauteil bereitgestellt wird;
b) ein Aufnahmebehälter bereitgestellt wird;
c) eine Kolbenstange bereitgestellt wird;
d) ein Vorschubmechanismus bereitgestellt wird;
e) eine Dosiervorrichtung bereitgestellt wird;
f) eine Anzeige bereitgestellt wird;
g) ein Auslösemechanismus bereitgestellt wird;
h) evtl. elektronische Bestandteile bereitgestellt werden;
i) eine technische Vorrichtung gemäß Anspruch 17 bereitgestellt wird;
j) die Einzelbestandteile aus a) bis i) zu einer funktionellen Einheit zusammen gebaut werden.

## Claims

1. A method for determining the position of a stopper of a medicine cartridge in a medical apparatus along a travel distance by means of
a) a light source and
b) a holder which fixes the stopper movably along a travel distance and
c) a photosensitive sensor surface,
which comprises initially generating a silhouette of the stopper on the sensor surface by irradiating the stopper with light from a), then the data relating to the silhouette being converted by a data processing unit into the position of the stopper along the travel distance wherein a differentiation is carried out depending on the stopper position:
- in which, in a first region at a start position, the stopper position is calculated on the basis of the position of the front edge,
- in which, in a second region, the stopper position is calculated from the position of the front edge and rear edge, and
- in which, in a third region, the stopper position is calculated on the basis of the position of the rear edge.

2. The method as claimed in claim 1, wherein the position of the stopper is in an insulin cartridge.

3. The method as claimed in claim 1 to 2, wherein a data processing unit is integrated in the medical apparatus.

4. The method as claimed in claim 1 to 2, wherein a separate data processing unit is operated together with the medical apparatus.

5. The method as claimed in one or more of claims 1 to 4, wherein an aperture is attached between a) and b) and/or b) and c).

6. The method as claimed in one or more of claims 1 to 5, wherein the light source consists of an LED row.

7. The method as claimed in claim 6, wherein the LED row gives a diffuse beam.

8. The method as claimed in claim 6, wherein the individual LEDs of the LED row have a small aperture angle.

9. The method as claimed in claim 6, wherein a converging lens is inserted between LED row and stopper.

10. The method as claimed in claim 9, where the converging lens is a cylindrical lens.

11. The method as claimed in claim 1 to 10, wherein the light source generates red light.

12. The method as claimed in one or more of claims 1 to 5, wherein laser light is generated by at least two light sources aligned side by side.

13. The method as claimed in claim 12, wherein red laser light is generated.

14. The method as claimed in one or more of claims 1 to 13, wherein the sensor surface consists of a row of arranged sensor elements.

15. The method as claimed in claim 14, wherein the sensor elements consist of a CCD line-scan camera.

16. The method as claimed in claim 14 or 15, wherein the sensitivity is greatest with red light.

17. A device for carrying out a method as claimed in one or more of claims 1 to 16, this device comprising at least
a) a light source; and
b) a holder which fixes a stopper movably along a travel distance; and
c) a photosensitive sensor surface and
d) a data processing unit.

18. The use of a device as claimed in claim 17 for assembling a medical apparatus which is suitable for administering a pharmaceutical into the human or animal body avoiding the gastrointestinal tract.

19. The use of a technical device as claimed in claim 18, wherein the pharmaceutical is insulin.

20. A medical apparatus for injecting a pharmaceutical into the human or animal body, the medical apparatus comprising inter alia
a) a base element for mounting at least one technical component;
b) a technical component in the form of a receptacle for a pharmaceutical;
c) a technical component in the form of a feed mechanism;
d) a technical component in the form of a metering device;
e) a technical component in the form of a display;
f) a technical component in the form of a release mechanism for starting up and carrying out the injection;
which additionally comprises at least one device as claimed in claim 17.

21. The medical apparatus as claimed in claim 20, wherein it is in the form and function of an insulin pen.

22. The medical apparatus as claimed in claim 20 or 21, which comprises at least one means for storing and/or processing data and/or signals, and at least one interface for transmission of data and/or signals to and/or from an external technical unit which is configured for the storage and/or processing of data and/or signals.

23. The medical apparatus as claimed in claim 22, wherein the external technical unit consists of a PC on which a program for the storage and/or processing of data and/or signals is installed.

24. The medical apparatus as claimed in one or more of claims 20 to 23, for injecting insulin.

25. The medical apparatus as claimed in claim 24, in which the insulin is a long-acting and/or a short-acting insulin.

26. The medical apparatus as claimed in one or more of claims 20 to 23, for injecting GLP-1.

27. The medical apparatus as claimed in one or more of claims 20 to 23, for injecting Lovenox.

28. The production of a medical apparatus as claimed in one or more of claims 20 to 27 for injecting a pharmaceutical into the human or animal body,
where
a) a base element is provided for mounting at least one technical component;
b) a receptacle is provided;
c) a plunger stem is provided;
d) a feed mechanism is provided;
e) a metering device is provided;
f) a display is provided;
g) a release mechanism is provided;
h) possibly electronic constituents are provided;
i) a technical device as claimed in claim 17 is provided;
j) the individual constituents from a) to i) are assembled to give a functional unit.

## Revendications

1. Procédé pour déterminer la position d'un bouchon d'une ampoule de médicament dans un appareil médical le long d'un trajet, à l'aide
a) d'une source de lumière, et
b) d'un arrêt, qui fixe le bouchon, avec possibilité de déplacement, le long d'un trajet, et
c) d'une surface de détection, sensible à la lumière, **caractérisé en ce qu'**on produit d'abord, par exposition du bouchon à la lumière de a), une silhouette du bouchon sur la surface de détection, puis les données concernant la silhouette sont, par une unité de traitement de données, converties en la position du bouchon le long du trajet, une distinction de cas étant effectuée en fonction de la position du bouchon :
- dans laquelle, dans une première région au niveau d'une position de départ, la position du bouchon est calculée à l'aide de la position du bord avant,
- dans laquelle, dans une deuxième région, la position du bouchon est calculée à partir de la position du bord avant et du bord arrière, et
- dans laquelle, dans une troisième région, la position du bouchon est calculée à l'aide de la position du bord arrière.

2. Procédé selon la revendication 1, **caractérisé en ce que** la position du bouchon se trouve dans une ampoule d'insuline.

3. Procédé selon les revendications 1 à 2, **caractérisé en ce qu'**une unité de traitement des données est intégrée dans l'appareil médical.

4. Procédé selon les revendications 1 à 2, **caractérisé en ce qu'**une unité de traitement des données distincte est utilisée en même temps que l'appareil médical.

5. Procédé selon l'une ou plusieurs des revendications 1 à 4, **caractérisé en ce qu'**un diaphragme est disposé entre a) et b) et/ou entre b) et c).

6. Procédé selon l'une des revendications 1 à 5, **caractérisé en ce que** la source de lumière est constituée d'une ligne de LED.

7. Procédé selon la revendication 6, **caractérisé en ce que** la ligne de LED rayonne d'une manière diffuse.

8. Procédé selon la revendication 6, **caractérisé en ce que** les LED individuelles de la ligne de LED présentent un petit angle d'ouverture.

9. Procédé selon la revendication 6, **caractérisé en ce qu'**une lentille collectrice est insérée entre la ligne de LED et le bouchon.

10. Procédé selon la revendication 9, dans lequel la lentille collectrice est une lentille cylindrique.

11. Procédé selon les revendications 1 à 10, **caractérisé en ce que** la source de lumière produit une lumière rouge.

12. Procédé selon l'une ou plusieurs des revendications 1 à 5, **caractérisé en ce que** la lumière laser est produite par au moins deux sources de lumière orientées l'une à côté de l'autre.

13. Procédé selon la revendication 12, **caractérisé en ce que** c'est une lumière laser rouge qui est produite.

14. Procédé selon l'une des revendications 1 à 13, **caractérisé en ce que** la surface de détection est constituée d'une série d'éléments capteurs rangés.

15. Procédé selon la revendication 14, **caractérisé en ce que** les éléments capteurs sont constitués d'une caméra CCD à balayage linéaire.

16. Procédé selon la revendication 14 ou 15, **caractérisé en ce que** la sensibilité est maximale en lumière rouge.

17. Dispositif pour la mise en oeuvre d'un procédé selon l'une ou plusieurs des revendications 1 à 16, ce procédé contenant au moins :
a) une source de lumière ; et
b) un arrêt, qui fixe un bouchon, avec possibilité de déplacement, le long d'un trajet ; et
c) une surface de détection sensible à la lumière, et
d) une unité de traitement des données.

18. Utilisation d'un dispositif selon la revendication 17 pour assembler un appareil médical convenant à l'administration d'un médicament dans le corps humain ou animal, en évitant le tractus gastro-intestinal.

19. Utilisation d'un dispositif technique selon la revendication 18, **caractérisée en ce que** le médicament est l'insuline.

20. Appareil médical pour injection d'un médicament dans le corps humain ou animal, l'appareil médical comprenant entre autres :
a) un corps de base pour montage d'au moins un composant technique ;
b) un composant technique sous forme d'un réservoir pour un médicament ;
c) un composant technique sous forme d'un mécanisme de poussée ;
d) un composant technique sous forme d'un dispositif doseur ;
e) un composant technique sous forme d'un affichage ;
f) un composant technique sous forme d'un mécanisme de déclenchement pour lancer et mettre en oeuvre l'injection ;
**caractérisé en ce qu'**il contient en outre au moins un dispositif selon la revendication 17.

21. Appareil médical selon la revendication 20, **caractérisé en ce qu'**il se présente sous la forme et avec la fonction d'un stylo à insuline.

22. Appareil médical selon la revendication 20 ou 21, **caractérisé en ce qu'**il contient au moins un moyen pour stocker et/ou traiter des données et/ou des signaux, ainsi qu'au moins une interface pour le transfert de données et/ou de signaux vers une unité technique externe et/ou à partir de cette dernière, qui est conçue pour le stockage et/ou le traitement de données et/ou de signaux.

23. Appareil médical selon la revendication 22, **caractérisé en ce que** l'unité technique externe est constituée d'un PC, sur lequel est installé un programme pour le stockage et/ou le traitement de données et/ou de signaux.

24. Appareil médical selon l'une ou plusieurs des revendications 20 à 23, pour l'injection d'insuline.

25. Appareil médical selon la revendication 24, dans lequel l'insuline est une insuline à action lente et/ou une insuline à action rapide.

26. Appareil médical selon l'une ou plusieurs des revendications 20 à 23, pour l'injection de GLP-1.

27. Appareil médical selon l'une ou plusieurs des revendications 20 à 23, pour l'injection de Lovenox.

28. Fabrication d'un appareil médical selon l'une ou plusieurs des revendications 20 à 27, pour l'injection d'un médicament dans le corps humain ou animal, dans lequel
a) on met à disposition un corps de base pour le montage d'au moins un composant technique ;
b) on met à disposition un réservoir ;
c) on met à disposition une tige de piston ;
d) on met à disposition un mécanisme de poussée ;
e) on met à disposition un dispositif doseur ;
f) on met à disposition un affichage ;
g) on met à disposition un mécanisme de déclenchement ;
h) éventuellement, on met à disposition des composants électroniques ;
i) on met à disposition un dispositif technique selon la revendication 17 ;
j) on assemble les éléments individuels de a) à i) pour obtenir une unité fonctionnelle.
